# EUROPÄISCHE PATENTSCHRIFT

(11) **EP 1 286 647 B1**
(45) Veröffentlichungstag und Bekanntmachung des Hinweises auf die Patenterteilung: **09.01.2008**
(21) Anmeldenummer: 01960245.7
(22) Anmeldetag: 25.05.2001
(51) Int. Cl.: A61K 8/49, A61Q 5/10

(54) **VERFAHREN ZUR OXIDATIVEN FÄRBUNG KERATINISCHER FASERN**
OXIDATION DYEING METHOD FOR KERATINOUS FIBERS
PROCEDE DE COLORATION PAR OXYDATION DE FIBRES DE KERATINE

(30) Priorität: 06.06.2000 DE 10027975; 27.04.2001 DE 10120807
(43) Veröffentlichungstag der Anmeldung: 05.03.2003
(73) Patentinhaber: Henkel Kommanditgesellschaft auf Aktien, 40589 Düsseldorf (DE)
(72) Erfinder: AKRAM, Mustafa, 22457 Hamburg (DE); RATH, Susanne, 20359 Hamburg (DE); HÖFFKES, Horst, 40595 Düsseldorf (DE)
(86) Internationale Anmeldenummer: PCT/EP2001/006016
(87) Internationale Veröffentlichungsnummer: WO 2001/093818

(56) Entgegenhaltungen:
- WO-A-93/09759
- WO-A-99/66890
- DE-A- 19 941 450
- FR-A- 2 649 887

## Beschreibung

Die vorliegende Erfindung betrifft Mittel zur oxidativen Färbung keratinischer Fasern enthaltend Indol- und/oder Indolinderivate und Pyrimidinderivate, sowie deren Verwendung als Färbemittel.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffinoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbexnittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Häufig sind aber in der Praxis - insbesondere bei den Modenuancen im Rotbereich - auch bei den oxidativen Färbungen unerwünschte Nuancenverschiebungen durch äußere Einflüsse wie beispielsweise Licht, Shampoonieren und Schweiß zu beobachten.

Es besteht daher nach wie vor die Aufgabe, oxidative Färbungen - insbesondere im Bereich der roten Modenuancen - hinsichtlich ihrer Echtheitseigenschaften zu verbessern. Bereits die Überwindung der Nachteile hinsichtlich einer dieser Eigenschaften stellt eine deutliche Verbesserung des Standes der Technik dar.

Es wurde nun überraschenderweise gefunden, daß diese Nachteile des Standes der Technik mit einem speziellen Färbemittel überwunden werden können.

Ein erster Gegenstand der vorliegenden Erfindung ist daher ein Mittel zur Färbung keratinischer Fasern, das
(A) mindestens ein Indol- und/oder Indolinderivat und
(B) mindestens eine Pyrimidinderivat*
enthält,

Unter keratinischen Fasern werden erfindungsgemäß Pelze, Wolle, Federn und insbesondere menschliche Haare verstanden.

In der internationalen Patentanmeldung WO-A1-93/09759 wird die Verwendung von speziellen Indolinderivaten als Farbstoffkomponente zusammen mit bekannten dizektziehenden Farbstoffen oder mit Oxidationsfarbstoffvorprodukten vom Kuppler- und Entwicklertyp zur Erzeugung von Färbungen beschrieben. Dieser Schrift sind aber keinerlei Hinweise auf Pyrimidinderivate zu entnehmen.

In einer ersten Ausführungsform der vorliegenden Erfindung enthält das Mittel als Komponente (A) mindestens ein Indolinderivat. Erfindungsgemäß bevorzugt sind Indolinderivate der Formel (Ia)
* und (C) als Kupplerkomponente mindestens ein Resorcinderivat 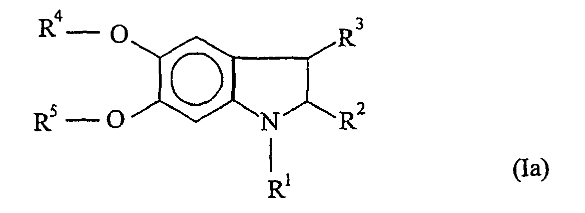 in der unabhängig voneinander
- R¹: steht für Wasserstoff, eine C₁-C₄-Alkylgruppe, eine C₁-C₄-Hydroxyalkylgruppe oder eine C₂-C₄-Polyhydroxyalkylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff oder eine C₁-C₄-Alkylgruppe,
- R⁴: steht für Wasserstoff, eine C₁-C₄-Alkylgruppe oder eine Gruppe -CO-R⁶, in der R⁶ steht für eine C₁-C₄-Alkylgruppe, und
- R⁵: steht für eine der unter R⁴ genannten Gruppen,
oder ein physiologisch verträgliches Salze dieser Verbindungen enthält.

Beispiele für die als Substituenten in den erfindungsgemäßen Verbindungen genannten C₁-bis C₄-Alkylgruppen sind die Gruppen Methyl, Ethyl, Propyl, Isopropyl und Butyl. Ethyl und Methyl sind bevorzugte Alkylgruppen. Bevorzugte C₁-C₄-Hydroxyalkylgruppen sind die Gruppen Hydroxymethyl, 2-Hydroxyethyl, 3-Hydroxypropyl oder 4-Hydroxybutyl; 2-Hydroxyethyl ist eine besonders bevorzugte Hydroxyalkylgruppe. Da es sich bei allen erfindungsgemäßen Substanzen um Amino-Verbindungen handelt, lassen sich aus diesen in üblicher Weise die bekannten Säureadditionssalze herstellen. Beispiele für solche Salze sind die Hydrochloride, die Hydrobromide, die Sulfate, die Phosphate, die Acetate, die Propionate, die Citrate und die Lactate.

Besonders bevorzugte Verbindungen der Formel (Ia) sind ausgewählt aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und 5,6-Dihydroxyindolin-2-carbonsäure. 5,6-Dihydroxyindolin ist eine ganz besonders bevorzugte Komponente der Formel (I).

Weiterhin sind aber auch die Indolinderivate 6-Hydroxyindolin, 6-Aminoindolin und 4-Aminoindolin erfindungsgemäß bevorzugt.

In einer zweiten Ausführungsform der vorliegenden Erfindung enthält das Mittel als Komponente (A) mindestens ein Indolderivat. Bevorzugt sind Indolderivate der Formel (Ib) 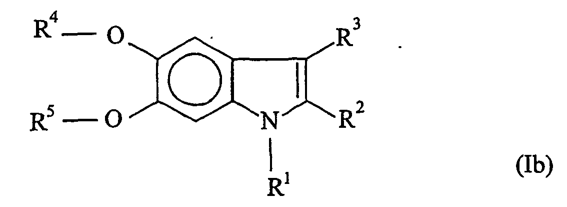 in der unabhängig voneinander
- R¹: steht für Wasserstoff, eine C₁-C₄-Alkylgruppe,0 eine C₁-C₄-Hydroxyalkylgruppe oder eine C₂-C₄-Polyhydroxyalkylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff oder eine C₁-C₄-Alkylgruppe,
- R⁴: steht für Wasserstoff, eine C₁-C₄-Alkylgruppe oder eine Gruppe -CO-R⁶, in der
- R⁶: steht für eine C₁-C₄-Alkylgruppe, und
- R⁵: steht für eine der unter R⁴ genannten Gruppen,
sowie eines der physiologisch verträglichen Salze dieser Verbindungen.

Beispiele für die als Substituenten in den erfindungsgemäßen Verbindungen genannten C₁bis C₄-Alkylgruppen sind die Gruppen Methyl, Ethyl, Propyl, Isopropyl und Butyl. Ethyl und Methyl sind bevorzugte Alkylgruppen. Bevorzugte C₁-C₄-Hydroxyalkylgruppen sind die Gruppen Hydroxymethyl, 2-Hydroxyethyl, 3-Hydroxypropyl oder 4-Hydroxybutyl; 2-Hydroxyethyl ist eine besonders bevorzugte Hydroxyalkylgruppe. Da es sich bei allen erfindungsgemäßen Substanzen um Amino-Verbindungen handelt, lassen sich aus diesen in üblicher Weise die bekannten Säureadditionssalze herstellen. Beispiele für solche Salze sind die Hydrochloride, die Hydrobromide, die Sulfate, die Phosphate, die Acetate, die Propionate, die Citrate und die Lactate.

Besonders bevorzugte Indolderivate der Formel (Ib) sind ausgewählt aus 5,6-Dihydroxyindol, N-Methyl-5,6-dihydroxyindol, N-Ethyl-5,6-dihydroxyindol, N.Propyl-5,6-dihydroxyindol, N-Butyl-5,6-dihydroxyindol und 5,6-Dihydroxyindol-2-carbonsäure. 5,6-Dihydroxyindol ist ein ganz besonders bevorzugtes Indolderivat der Formel (Ib).

Weiterhin haben sich auch die Indolderivate 6-Hydroxyindol, 6-Aminoindol und 4-Aminoindol als er.6ndungsgemäß geeignet erwiesen.

In einer weiteren Ausführungsform der vorliegenden Erfindung kann das Mittel als Komponente (A) mindestens ein Indolderivat und mindestens ein Indolinderivat enthalten.

Weiterhin enthält das erfindungsgemäße Mittel als Komponente (B) mindestens ein Pyrimidinderivat,

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethyl-amino-4,5,6-triaminopyrimidin, 2,4-Dihydroxy-5,6-diaminopyrimidin und 2,5,6-Triamino-Pyrimidin. 2,4,5,6-Tetraaminopyrimidin, 2-Dimethylamino-4,5,6-triaminopyrimidin, 2-Hydroxy-4,5,6-triaminopyrimidin und 4-Hydroxy-2,5,6-triaminopyrimidin sind bevorzugt. 2,4,5,6-Tetraaminopyrimidin ist ein ganz besonders bevorzugtes Pyrimidinderivat.

Das Resorcinderivat (C) ist vorzugsweise ausgewählt aus Resorcin, Resorcinmonomethylether,2-Methylresorcin, 5-Methylresorcin, 2,5-Dimethylresorcin, 2-Chlorresorcin, 4-Chlorresorcin, Pyrogallol und 1,2,4-Trihydroxybenzol.

Neben den erfindungswesenthcheu Komponenten (A), (B) und (C) kann das Färbemittel erfindungsgemäß noch eine oder mehrere weitere Entwicklerkomponenten enthalten.

Es kann erfindungsgemäß bevorzugt sein, als weitere Entwicklerkomponente ein p-Phenylendiaminderivat oder eines seiner physiologisch verträglichen Salze einzusetzen. Besonders bevorzugt sind p-Phenylendiami.nderivate der Formel (E1) 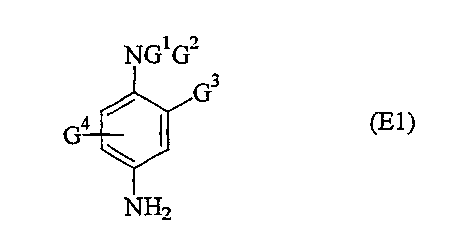 wobei
- G¹ steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁-bis C₄)-alkylradikal, ein 4'-Aminophenylradikal oder ein C₁- bis C₄-Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁-bis C₄)-alkykadikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jod-oder Fluoratom, ein C₁- bis C₄-Alkylradikal, ein C₁- bis C₄-Monohydroxyalkylradikal, ein - C₂- bis C₄-Polyhydroxyalkylradikal, ein C₁- bis C₄-Hydroxyalkoxyradikal, ein C₁- bis C₄-Acetylaminoalkoxyradikal, ein C₁- bis C₄- Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N -diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β-γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino-und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 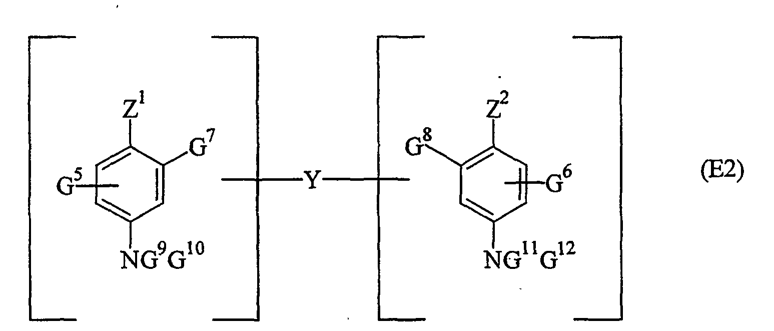 wobei:
- Z¹ und Z² stehen unabhängig voneinander für ein Hydroxyl- oder NH₂-Radikal, das gegebenenfalls durch ein C₁- bis C₄-Alkylradikal, durch ein C₁- bis C₄-Hydroxyalkylradikal und/oder durch eine Verbrückung Y substituiert ist oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈-Alkoxyradikale substituiert sein kann, oder eine direkte Bindung,
- G⁵ und G⁶ stehen unabhängig voneinander für ein Wasserstoff- oder Halogenatom, ein C₁- bis C₄-Alkylradikal, ein C₁- bis C₄-Monohydroxyalkykadikal, ein C₂- bis C₄- Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder ein C₁- bis C₄-Alkykadikal, mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-bis-(β-Hydroxyethyl)-N,N'-bis-(4'-aminophenyl)-1,3-diamino-propan-2-ol, N,N'-bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis-(4'-amino-3'-methylphenyl)-ethylendiamin, Bis-(2-hydroxy-5-aminophenyl)-methan, 1,4-Bis-(4'-aminophenyl)-diaza-cycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-bis-(β-Hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 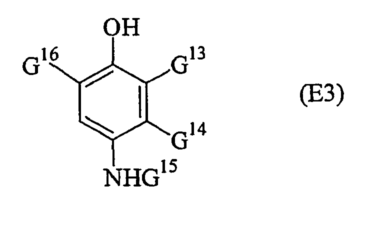 wobei:
- G¹³ steht für ein Wasserstoffatom, ein Halogenatom, ein C₁- bis C₄-Alkylradikal, ein C₁- bis C₄.Monohydroxyalk-ylradikal, ein C₂- bis C₄-Polyhydroxyalkylradikal, ein (C₁-bis C₄).Akoxy-(C₁- bis C₄)-alkykadikal, ein C₁- bis C₄-Aminoalkylradikal, ein Hydroxy-(C₁- bis C₄)-alkylaminoradikal, ein C₁- bis C₄-Hydroxyalkoxyradikal, ein C₁- bis C₄-Hydroxyalkyl-(C₁-bis C₄)-aminoalkylradikal oder ein (Di-C₁- bis C₄-Alkylamino)-(C₁- bis C₄)-alkylradikal, und
- G¹⁴ steht für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkykadikal, ein C₁- bis C₄-Aminoalkylradikal oder ein C₁- bis C₄-Cyanoalkylradikal,
- G¹⁵ steht für Wasserstoff, ein C₁ bis C₄-Alkylradikal, ein C₁- bis C₄-Monohydroxyalkylradikal, ein C₂- bis C₄-Polyhydroxyallcylradikal, ein Phenylradikal oder ein Benzylradikal, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethylaminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(B-Methoxyethyl)amino-3-amino-6-methoxy-pyridin und 3,4-Diamino-pyridin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-iso-propylpyrazol, 4-Amino-5-(2'-aminoethyl)amino-1 ,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 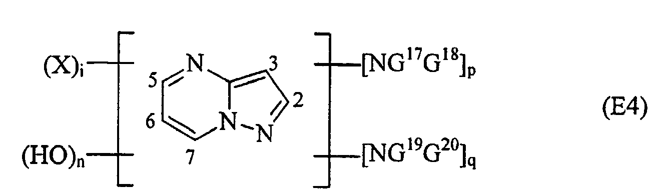 wobei:
- G¹⁷, G¹⁸, G¹⁹ und G²⁰, unabhängig voneinander stehen für ein Wasserstoffatom, ein C₁- bis C₄-Alkylradikal, ein Aryl-Radikal, ein C₁- bis C₄-Hydroxyalkylradikal, ein C₂- bis C₄-Polyhydroxyalkylradikal ein (C₁- bis C₄)-Alkoxy-(C₁- bis C₄)-alkylradikal, ein C₁- bis C₄-Aminoalkylradikal, das gegebenenfalls durch ein Acetyl-Ureid- oder Sulfonyl-Radikal geschützt sein kann, ein (C₁- bis C₄)-Alkylamino-(C₁- bis C₄)-alkylradikal, ein Di-[(C₁-bis C₄)-alkyl]-(C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄)-[Hydroxyalkyl]-(C₁- bis C₄)-amino-alkylradikal,
- die X-Radikale stehen unabhängig voneinander für ein Wasserstoffatom, ein C₁-bis C₄-Alkylradikal, ein Aryl-Radikal, ein C₁- bis C₄-Hydroxyalkyladikal, ein C₂- bis C₄-Polyhydroxyalkylradikal, ein C₁- bis C₄-Aminoalkylradikal, ein (C₁- bis C₄)-Alkylamino-(C₁- bis C₄)-alkylradikal, ein Di-[(C₁- bis C₄)alkyl]- (C₁- bis C₄)-aminoalkylradikal,
wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄-hy-droxyalkyl)-aminoalkylradikal, ein Aminoradikal, ein C₁- bis C₄-Alkyl-oder Di-(C₁- bis C₄-hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1, mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG17GI8 (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

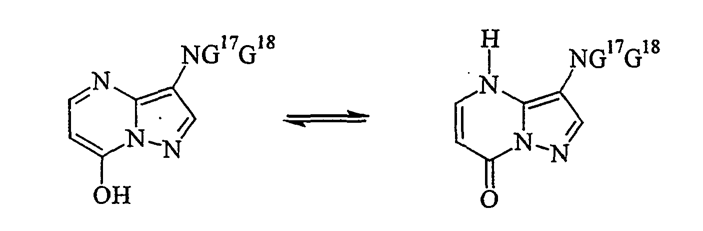

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-[1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pynmidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5a]-pyrimidin-3,7-diamin;
sowie ihre physiologisch verträglichen Salze und ihre tautomeren Formen, wenn ein tautomeres Gleichgewicht vorhanden ist

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Weiterhin kann das erfindungsgemäße Mittel mindestens ein weitere Kupplerkomponente enthalten. Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Di-hydroxy-5-(ethylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, l,3-Bn-(2,4-diaininophenoxy)-propan, 1 -Methoxy-2-amino-4-(2'-hydroxyethylamino)benzol, 1,3- Bis-(2,4-diaminophenyl)-propan, 2,6-Bis-(2-hydroxyethylamino)-1-methylbenzol und 1-Amino-3-bis-(2-hydroxyethyl)-aminobenzol,
- o-Diaminobenzol und dessen Derivate wie beispielsweise 3,4-Diaminobenzoesäure und 2,3-Diamino-1-methylbenzol,
- Pyridinderivate wie beispielsweise 2,6-Dihydroxypyridin, 2-Amino--3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1 naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-di-hydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Di-hydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugt sind Kupplerkomponenten vom m-Aminophenoltyp. Weiterhin sind 1 -Naphthol, 1,5-, 2,7- und 1,7-Dihydroxynaphthalia, 3-Aminophenol, 5-Amino-2-methylphenol, 2-Amino-3-hydroxypyridin, Resorcin, 2- Chlor-6-methyl-3-aminophenol, und 2,6-Dihydroxy-3,4-dimethylpyridin erfindungsgemäß bevorzugte Kupplerkomponenten.

In einer besonders bevorzugten Ausführungsform wird als Kupplerkomponente 2-Methylresorcin eingesetzt. Diese Kupplerkomponente eignet sich besonders als Kupplerkomponente zur Erzielung von Nuancen im roten Bereich mit Pyrimidinderivaten.
Ferner können die erfindungsgemäßen Mittel einen oder mehrere direktziehende Farbstoff enthalten. Direktziehende Farbstoffe sind üblicherweise Nitrophenylendiamine, Nitroaminophenole, Azofarbstoffe, Anthrachinone oder Indophenole.

Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1 -hydroxy-4-nitrobenzol.

Ferner können die erfindungsgemäßen Mittel einen kationischen direktziehenden Farbstoff enthalten. Besonders bevorzugt sind dabei
- (C1): kationische Triphenylmethanfarbstoffe, wie beispielsweise Basic Blue 7, Basic Blue 26, Basic Violet 2 und Basic Violet 14,
- (C2): aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
- (C3): direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (C3) sind insbesondere die folgenden Verbindungen:

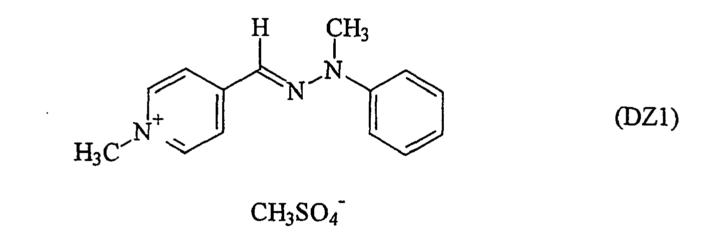

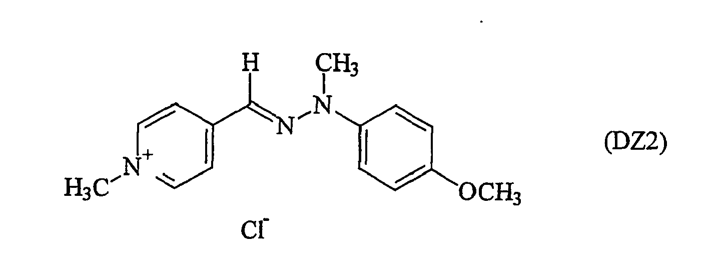

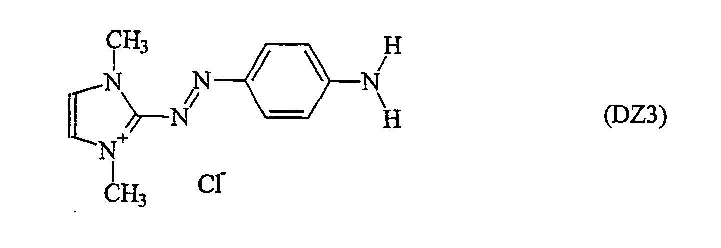

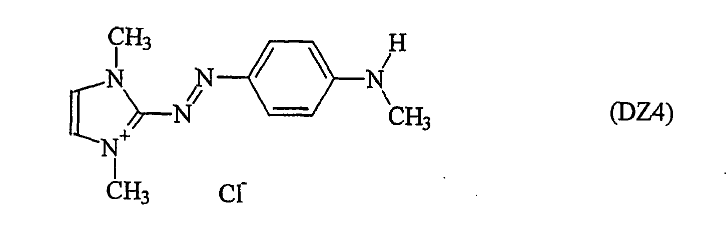

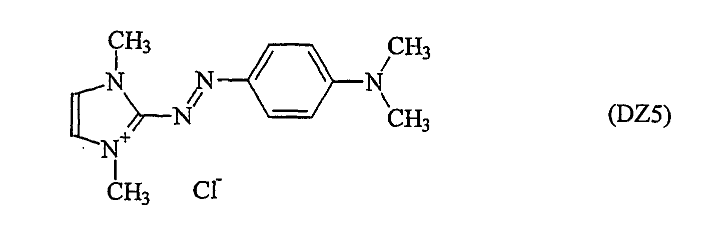

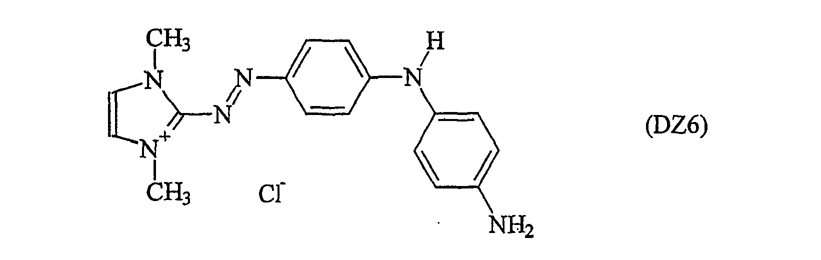

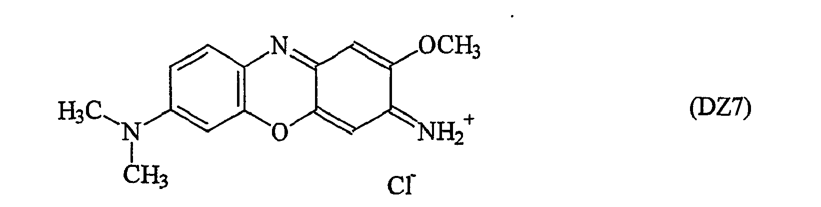

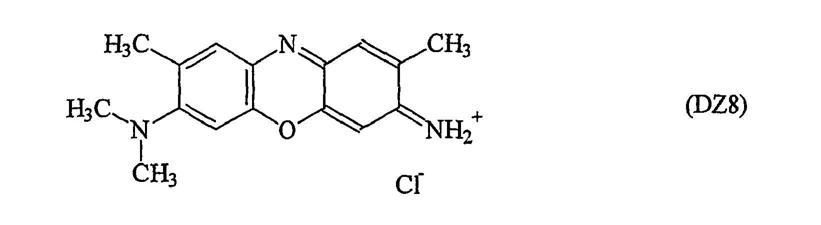

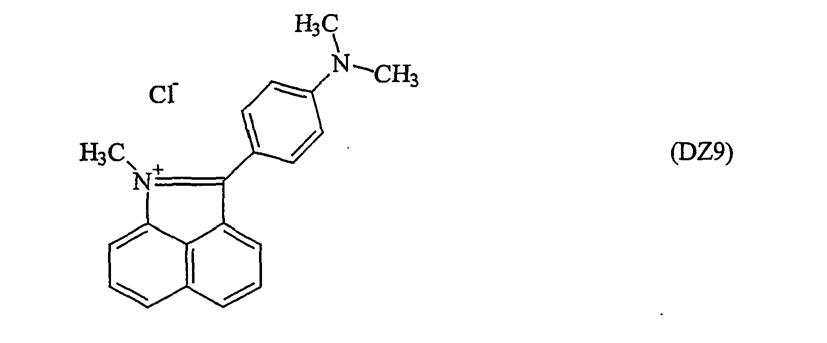

Die Verbindungen der Formeln (DZ1), (DZ3) und (DZ5) sind ganz besonders bevorzugte kationische direktziehende Farbstoffe der Gruppe (C3).

Die erfindungsgemäßen Mittel enthalten die direktziehenden Farbstoffe bevorzugt in einer Menge von 0,01 bis 20 Gew.-%, bezogen auf das gesamte Färbemittel.

Die erfindungsgemäßen Mittel enthalten die Wirkstoffe bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Die eigentliche Anwendungszubereitung wird daher zweckmäßigerweise unmittelbar vor der Anwendung durch Mischung der Zubereitung des Oxidationsmittels mit dem erfindungsgemäßen Mittel hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂₋Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethyl-ammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethyl-ammoniumchlorid, sowie Dehyquaft^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCl-Nomenklatur ein "Lauryl Methyl Gluceth-10 Hydroxypropyl Dimonium Chloride".

Weiterhin bevorzugte kationische Tenside, die auch pflegende Eigenschaften aufweisen, sind die Verbindungen der Formel (II)

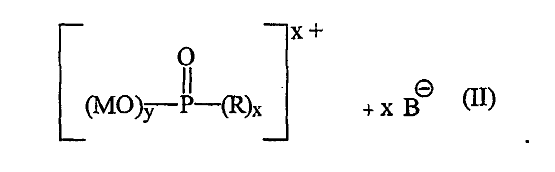

In der Formel (II) steht y für eine ganze Zahl von 0 bis 2, x für eine ganze Zahl von 1 bis 3 mit der Maßgabe, daß die Summe aus x und y gleich 3 ist.

In den Verbindungen der Formel (II) steht ferner M für Wasserstoff, ein Äquivalent eines Alkali- oder Erdalkalimetallkations, ein Ammoniumkation oder einen Alkylrest mit 1 bis 4 Kohlenstoffatomen, der gegebenenfalls mit einer oder mehreren Hydroxygruppe(n) substituiert ist. Besonders bevorzugt sind Verbindungen, bei denen M für ein Natriumkation steht.

Weiterhin steht B in der Formel (II) der erfindungsgemäß einzusetzenden Tenside für ein Äquivalent eines physiologisch verträglichen Anions. Als Anion eignen sich beispielsweise Chlorid, Bromid, Jodid, Sulfat, Perchlorat, Tetrafluorborat, Tetraphenyl-borat und Tetrachlorozinkat. Bevorzugt ist das Chloridion.

R steht in den erfindungsgemäßen Tensiden der Formel (II) für einen Rest der Formel (III), 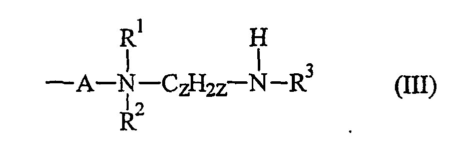 in der z für eine ganze Zahl von 1 bis 4, insbesondere für 3, steht und
R¹ und R² unabhängig voneinander für einen C₁- bis C₄-Alkylrest stehen, der gegebenenfalls mit einer oder mehreren Hydroxygruppe(n) oder einer Acylgruppe substituiert ist.

A steht erfindungsgemäß für eine der Einheiten -O-CH₂-CH₂-CH₂-, -O-CH₂-CH₂- oder -O-CH₂-CHOH-CH₂-, wobei die Einheit -O-CH₂-CHOH-CH₂- besonders bevorzugt ist.

Der Rest R³ steht für
(a) einen verzweigten oder unverzweigten, gesättigten C₈- bis C₁₈-Acylrest oder
(b) einen verzweigten oder unverzweigten, einfach oder mehrfach ungesättigten C₈- bis C₁₈-Acylrest.

Besonders bevorzugte gesättigte Reste R³ sind der Rest der Stearinsäure sowie die Reste der Mischung der Fettsäuren, die man aus Kokosöl gewinnt.

Ein besonders bevorzugter ungesättigter Rest R³ ist der Rest der Linolsäure. Überraschenderweise wurde gefunden, daß sich Verbindungen der Formel (II), bei denen R³ der Rest der Linolsäure ist, durch eine höhere Verträglichkeit mit Emulgatorsystemen auszeichnen. Dies bedeutet, daß sich diese Substanzen leichter in die Formulierungen einarbeiten lassen. Weiterhin weisen Formulierungen mit Verbindungen der Formel (II), bei denen R³ für den Rest der Linolsäure steht, einen deutlich höheren Pflegeeffekt im Vergleich zu Verbindungen mit gesättigten Fettsäureresten auf.

Ganz besonders bevorzugte Verbindungen der Formel (II) sind die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalirnetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die im Rahmen des erfindungsgemäßen Verfahrens eingesetzten Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vylpyrrolidon-Methoimidazoliniumchlorid-Copolymere, wie sie unter der Bezeichnung Luviquat^{®} angeboten werden.
- quaternierter Polyvinylalkohol sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl-und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der vorliegenden Erfindung ist die Verwendung eines erfindungsgemäßen Mittels zur Färbung keratinischer Fasern.

Eine bevorzugte Ausführungsform dieses Gegenstandes ist die Verwendung eines erfindungsgemäßen Mittels zur Erzielung intensiver Färbungen - insbesondere im Bereich der roten Modenuancen - mit einer hohen Waschechtheit.

Ein dritter Gegenstand der vorliegenden Erfindung ist ein Verfahren zur Färbung keratinischer Fasern, bei dem ein erfindungsgemäßes Mittel auf die Fasern aufgetragen und nach einer Einwirkungszeit abgespült wird.

In einer bevorzugten Ausführungsform enthält das Mittel, das im Rahmen des erfindungsgemäßen Verfahrens auf die Fasern aufgetragen wird, weiterhin mindestens ein Oxidationsmittel, insbesondere Wasserstoffperoxid.

### Ausführungsbeispiele

Die folgenden Angaben sind, soweit nichts anderes vermerkt ist, in Gewichtsprozent.

### Beispiel 1:

| Komponente | Vergleichsrezeptur V1 | Erfindungsgemäße Rezeptur E1 |
|---|---|---|
| Cetylalkohol | 10,00 | 10,00 |
| Cutina^{®} CP-V¹ | 3,00 | 3,00 |
| Emulgator 157² | 2,00 | 2,00 |
| Rewoamid^{®} IPP 240³ | 10,00 | 10,00 |
| Stearinsäure | 5,00 | 5,00 |
| Wollwachsalkohol | 4,00 | 4,00 |
| Phospholipid^{®} EFA⁴ | 0,10 | 0,10 |
| Titandioxid | 1,00 | 1,00 |
| Disodium Cocoamphodiacetat | 2,00 | 2,00 |
| Tetrasoduim EDTA | 0,15 | 0,15 |
| Ascorbinsäure | 0,10 | 0,10 |
| Natriumsulfit | 0,50 | 0,50 |
| Mirapol^{®} A-15⁵ | 0,10 | 0,10 |
| 2,4,5,6-Tetraaminopyridin | 1,50 | 1,50 |
| 2-Methylresorcin | 0,80 | 0,80 |
| p-Toluylendiamin | 0,30 | 0,30 |
| Resorcin | 0,10 | 0,10 |
| 2-Amino-3-hydroxypyridin | 0,05 | 0,05 |
| m-Aminophenol | 0,03 | 0,03 |
| HC Red BN⁶ | 0,50 | 0,50 |
| HC Red 3 | 0,30 | 0,30 |
| 5,6-Dihydroxyindolin | --- | 0,10 |
| Wasser | ad 100 | ad 100 |

| | | |
|---|---|---|
| ¹ C₁₆₋₁₈-Fettsäure-C₁₂-₁₈alkylester (INCI-Bezeichnung: Cetyl Palmitate) (Cognis) ² Mischung von Mono- und Distearat des Propylen-1,2-glykols (INCI-Bezeichnung: Propylene Glycol Stearate SE) (Th. Goldschmidt) ³ Kokosfettsäuremonoisopropanolamid (INCI-Bezeichnung: Cocamide Mipa) (Goldschmidt-Rewo) ⁴ ca. 30% Aktivsubstanz; (INCI-Bezeichnung: Linoleamidopropyl PG-Dimonium Chloride Phosphate) (Mona) ⁵ ca. 64% Aktivsubstanz (INCI-Bezeichnung: Polyquaternium-2) (Rhodia) ⁶ 3-(4'-Hydroxy-2'-nitroanilino)-propanol | | |

Die Formulierungen V1 und E1 wurden im Halbseitentest auf den Köpfen von 10 Probanden getestet. Dazu wurden 60g der jeweiligen Färbecreme mit 60g einer 6%igen wäßrigen Wasserstoffperoxidzubereitung gemischt und die resultierenden Anwendungszubereitungen jeweils auf die Fasern an einer Seite des Kopfes der Probanden aufgetragen. Nach einer Einwirkungszeit von 30min wurden die Haare gründlich gespült. Nach jeder Färbung und nach jeweils 3-, 6-, 12- und 18-maliger Haarwäsche wurde die Haarfärbung von 3 Friseuren visuell an beiden Seiten beurteilt. Dieser Vorgang wurde bei 3 aufeinanderfolgenden Färbungen im Abstand von 4 Wochen wiederholt.

Es zeigte sich durchweg eine intensivere Erstfärbung und ein deutlich verbesserter Farberhalt der Formulierungen mit Zusatz von 5,6-Dihydroxyindolin (E1). Der Farbunterschied der Haarhälften der Probanden trat insbesondere nach mehreren Wäschen deutlich hervor.

### Beispiel 2:

| | |
|---|---|
| Ammoniumcarbopollsg. 1 %ig⁸ | 10,00 |
| Ammoniumrohagitlsg. 6%ig⁹ | 3,00 |
| Hycryl Mulgol^{®} 012¹⁰ | 4,50 |
| Kaliumoleinseife 12,5%ig | 6,50 |
| Kaliumrizinusseife 12,5%ig | 2,00 |
| Plantaren^{®} 2000 CS/UP¹¹ | 0,50 |
| Titandioxid | 0,20 |
| Cetiol^{®} V¹² | 2,50 |
| Cetylalkohol | 10,00 |
| Glycerinmonostearat NSE¹³ | 2,00 |
| Phospholipid EFA | 0,50 |
| Tetrasodium EDTA | 0,30 |
| Mirapol A-15 | 0,150 |
| Ascorbinsäure | 0,10 |
| Natriumsulfit | 0,10 |
| Ammoniak 25% ig | 4,00 |
| Parfüm | 0,50 |
| 2,4,5,6-Tetraaminopyrimidin | 1,50 |
| 2-Methylresorcin | 0,69 |
| HC Red BN | 0,50 |
| HC Red 3 | 0,3 |
| 5,6-Dihydroxyindolin | 0,25 |
| Wasser | ad 100 |

| | |
|---|---|
| ⁸ Lösung eines Ammoniumsalzes eines Methacrylsäure-Methylacrylat-Copolymeren (INCI-Bezeichnung: Ammonium Polyacrylate) (Röhm GmbH) ⁹ Lösung eines Ammoniumsalzes eines Acrylsäure-Polymeren (INCI-Bezeichnung: Ammonium Acrylates Copolymer) (Goodrich) ¹⁰ Oleylalkohol mit 10 EO-Einheiten (Chemische Fabrik Dr. Angele GmbH) ¹¹ C₈₋₁₆-Alkyl-1 ,4-Polyglucosid (ca. 51 % Aktivsubstanz; INCI-Bezeichnung: Decyl Glucoside) (Henkel) ¹² Ölsäuredecylester (INCI-Bezeichnung: Decyl Oleate) (Henkel) ¹³ (INCl-Bezeichnung: Glyceryl Strearate) (Oleofina) | |

60g der Färbecreme wurden mit 60g einer 6%igen wäßrigen Wasserstoffperoxidzubereitung gemischt und die resultierenden Anwendungszubereitung auf die Fasern aufgetragen. Nach einer Einwirkungszeit von 30min wurden die Haare gründlich gespült.

## Patentansprüche

1. Mittel zur Färbung keratinischer Fasern, **dadurch gekennzeichnet, dass** es
(A) mindestens ein Indol- und/oder Indolinderivat,
(B) mindestens ein Pyrimidinderivat und
(C) als Kupplerkomponente mindestens ein Resorcinderivat enthält.

2. Mittel nach Anspruch 1, **dadurch gekennzeichnet, dass** es als Komponente (A) mindestens ein Indolinderivat der Formel (1a) 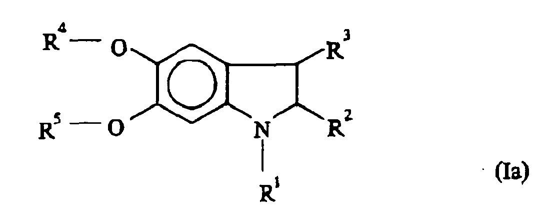 in der unabhängig voneinander
R¹ steht für Wasserstoff, eine C₁-C₄-Alkylgruppe, eine C₁-C₄-Hydroxyalkylgruppe oder eine C₂C₄-Polyhydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶. in der
R⁶ steht für eine C₁-C₄-Alkylgruppe, und
R⁶ steht für eine der unter R⁴ genannten Gruppen,
oder ein physiologisch verträgliches Salze dieser Verbindungen enthält.

3. Mittel nach Anspruch 2. **dadurch gekennzeichnet, dass** die Verbindung der Formel (1a) ausgewählt ist aus 5,6-Dihydroxyindolin, N-Methyl-5,6-dihydroxyindolin, N-Ethyl-5,6-dihydroxyindolin, N-Propyl-5,6-dihydroxyindolin. N-Butyl-5.6-dihydroxyindolin und 5,6-Dihydroxyindolin-2-carbonsäure.

4. Mittel nach Anspruch 3, **dadurch gekennzeichnet, dass** die Verbindung der Formel (Ia) 5,6-Dihydroxyindolin ist

5. Mittel nach einem der Ansprüche 1 bis 4, **dadurch gekennzeichnet, dass** es als Komponente (A) mindestens ein Indolderivat der Formel (1b) 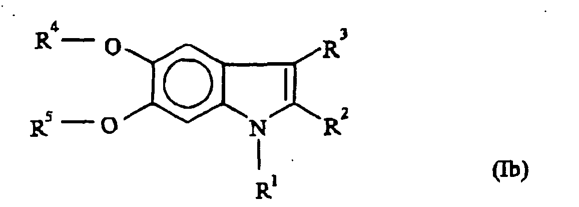 in der unabhängig voneinander
R¹ steht für Wasserstoff, eine C₁-C₄-Alkylgruppe, eine C₁-C₄-Hydroxyalkylgruppe oder eine C₂C₄-Polyhydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-AJkylgruppe,
R⁴ steht für Wasserstoff, eine C₁-C₄-AJkylgruppe oder eine Gruppe -CO-R⁸, in der
R⁸ steht für eine C₁-C₄-Alkylgruppe, und
R⁵ steht für eine der unter R⁴ genannten Gruppen.
sowie eines der physiologisch verträglichen Salze dieser Verbindung enthält.

6. Mittel nach Anspruch 5, **dadurch gekennzeichnet, dass** das Indolderivat der Formel (1b) ausgewählt ist aus 5,6-Dihydroxyindol, N-Methyl-5,6-dihydroxyindol. N-Ethyl-5,6-dihydroxyindol, N-Propyl-5,6-dihydroxyindol, N-Butyl-5,6-dihydroxyindol und 5,6-Dihydroxyindol-2-carbonsäure.

7. Mittel nach Anspruch 6, **dadurch gekennzeichnet, dass** das Indolderivat der Formel (1b) 5,6-Dihydroxyindol ist

8. Mittel nach einem der Ansprüche 1 bis 7, **dadurch gekennzeichnet, dass** das Pyrimidinderivat ausgewählt ist aus 2,4,5.6-Tetraaminopyrimidin, 2-Dimethylamino-4.5.6-triamirlopyrimidin, 2-Hydroxy-4.5.6-triaminopyrimidin und 4-Hydroxy-2,5,6-triaminopyrimidin.

9. Mittel nach Anspruch 8, **dadurch gekennzeichnet, dass** das Pyrimidinderivat 2,4,5,6-Tetraaminopyrlmidin ist.

10. Mittel nach einem der Ansprüche 1 bis 9, **dadurch gekennzeichnet, dass** es mindestens eine weitere Entwicklerkomponente enthält,

11. Mittel nach einem der Ansprüche 1 bis 10, **dadurch gekennzeichnet, dass** das Resorcinderivat ausgewählt ist aus Resorcin, Resordimonomethylether. 2-Methylresorcin, 5-Methylresorcin, 2,5-Dimethylresorcin, 2-Chlorresorcin. 4-Chlorresorcin, Pyrogallol und 1,2,4-Trihydroxybenzol.

12. Mittel nach Anspruch 11, **dadurch gekennzeichnet, dass** das Resorcinderivat 2-Methylresorcin ist.

13. Mittel nach einem der Ansprüche 1 bis 12, **dadurch gekennzeichnet, dass** es weiterhin mindestens einen direktziehenden Farbstoff enthält.

14. Verwendung eines Mittels nach einem der Ansprüche 1 bis 13 zu Färbung keratinischer Fasern

15. Verwendung nach Anspruch 14 zur Erhöhung der Waschechtheit der resultierenden Färbung - insbesondere im Bereich der Rotnuancen.

16. Verfahren zur Färbung keratinischer Fasern, bei dem ein Mittel, enthaltend
(A) mindestens ein Indol- und/oder Indolinderivat,
(B) mindestens ein Pyrimidinderivat und
(C) als Kupplerkomponente mindestens ein Resorcinderivat
auf die Fasern aufgetragen und nach einer Einwirkungszeit abgespült wird.

17. Verfahren nach Anspruch 16, **dadurch gekennzeichnet, dass** das Mittel weiterhin ein Oxidationsmittel, insbesondere Wasserstoffperoxid, enthält.

## Claims

1. Agent for dyeing keratinous fibres, **characterized in that** it comprises
(A) at least one indole derivative and/or indoline derivative,
(B) at least one pyrimidine derivative and
(C) as coupler component, at least one resorcinol derivative.

2. Agent according to Claim 1, **characterized in that** it comprises, as component (A), at least one indoline derivative of formula (Ia) 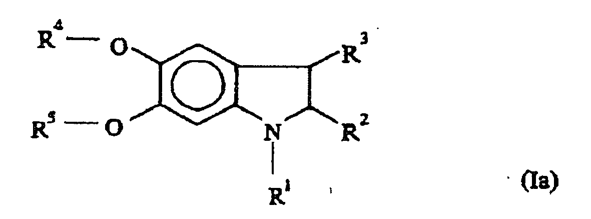 in which, independently of one another,
R¹ is hydrogen, a C₁-C₄-alkyl group, a C₁-C₄-hydroxyalkyl group or a C₂-C₄-polyhydroxyalkyl group,
R² is hydrogen or a -COOH group, where the -COOH group may also be in the form of a salt with a physiologically compatible cation,
R³ is hydrogen or a C₁-C₄-alkyl group,
R⁹ is hydrogen, a C₁-C₄-alkyl group or a group -CO-R ⁶ in which
R⁶ is a C₁-C₄-alkyl group, and
R⁵ is one of the groups specified under R⁴
or a physiologically compatible salt of these compounds.

3. Agent according to Claim 2, **characterized in that** the compound of formula (Ia) is selected from 5,6-dihydroxyindoline, N-methyl-5,6-dihydroxyindoline, N-ethyl-5,6-dihydroxyindoline, N-propyl-5,6-dihydroxyindoline, N-butyl-5,6-dihydroxyindoline and 5,6-dihydroxyindoline-2-carboxylic acid.

4. Agent according to Claim 3, **characterized in that** the compound of formula (Ia) is 5,6-dihydroxyindoline.

5. Agent according to one of Claims 1 to 4, **characterized in that** it comprises, as component (A), at least one indole derivative of formula (Ib) 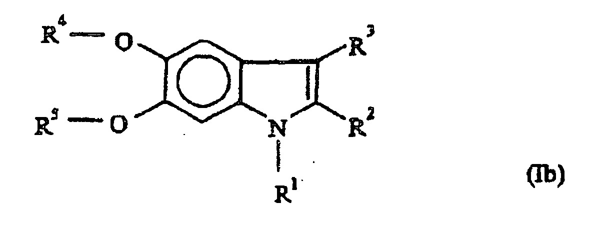 in which, independently of one another,
R¹ is hydrogen, a C₁-C₄-alkyl group, a C₁-C₄-hydroxyalkyl group or a C₂-C₄-polyhydroxyalkyl group,
R² is hydrogen or a -COOH group, where the -COOH group may also be in the form of a salt with a physiologically compatible cation,
R³ is hydrogen or a C₁-C₄-alkyl group,
R⁴ is hydrogen, a C₁-C₄-alkyl group or a group -CO-R ⁶ in which
R⁶ is a C₁-C₄-alkyl group, and
R⁵ is one of the groups specified under R ⁴
and one of the physiologically compatible salts of this compound.

6. Agent according to Claim 5, **characterized in that** the indole derivative of formula (Ib) is selected from 5,6-dihydroxyindole, N-methyl-5,6-dihydroxyindole, N-ethyl-5,6-dihydroxyindole, N-propyl-5,6-dihydroxyindole, N-butyl-5,6-dihydroxyindole and 5,6-dihydroxyindole-2-carboxylic acid.

7. Agent according to Claim 6, **characterized in that** the indole derivative of formula (Ib) is 5,6-dihydroxyindole.

8. Agent according to one of Claims 1 to 7, **characterized in that** the pyrimidine derivative is selected from 2,4,5,6-tetraaminopyrimidine, 2-dimethylamino-4,5,6-triaminopyrimidine, 2-hydroxy-4,5,6-triaminopyrimidine and 4-hydroxy-2,5,6-triaminopyrimidine.

9. Agent according to Claim 8, **characterized in that** the pyrimidine derivative is 2,4,5,6-tetraaminopyrimidine.

10. Agent according to one of Claims 1 to 9, **characterized in that** it comprises at least one further developer component.

11. Agent according to one of Claims 1 to 10, **characterized in that** the resorcinol derivative is selected from resorcinol, resorcinol monomethyl ether, 2-methylresorcinol, 5-methylresorcinol, 2,5-dimethylresorcinol, 2-chlororesorcinol, 4-chlororesorcinol, pyrogallol and 1,2,4-trihydroxybenzene.

12. Agent according to Claim 11, **characterized in that** the resorcinol derivative is 2-methylresorcinol.

13. Agent according to one of Claims 1 to 12, **characterized in that** it further comprises at least one direct dye.

14. Use of an agent according to one of Claims 1 to 13 for dyeing keratinous fibres.

15. Use according to Claim 14 for increasing the wash fastness of the resulting coloration - in particular in the range of red nuances.

16. Method for dyeing keratinous fibres, in which an agent comprising
(A) at least one indole derivative and/or indoline derivative,
(B) at least one pyrimidine derivative and
(C) as coupler component, at least one resorcinol derivative
is applied to the fibres and rinsed off after a contact time.

17. Method according to Claim 16, **characterized in that** the agent further comprises an oxidizing agent, in particular hydrogen peroxide.

## Revendications

1. Agent de teinture de fibres kératiniques, **caractérisé en ce qu'**il contient
(A) au moins un dérivé d'indole et/ou d'indoline
(B) au moins un dérivé de pyrimidine et
(C) comme composant de couplage au moins un dérivé de résorcinol.

2. Agent selon la revendication 1, **caractérisé en ce qu'**il contient comme composant (A) au moins un dérivé d'indoline répondant à la formule (1a) 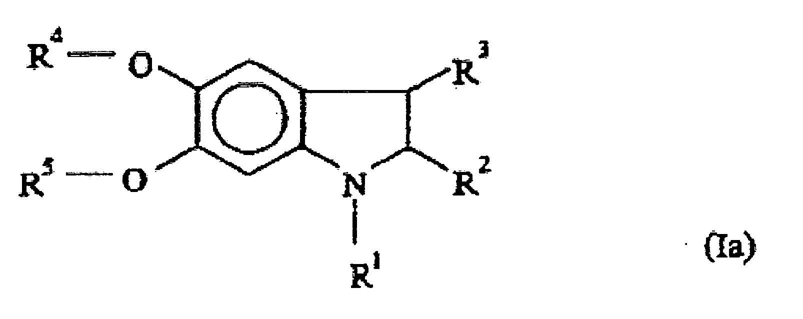 dans laquelle, indépendamment les uns des autres
R¹ représente un atome d'hydrogène, un groupe alkyle en C₁-C₄, un groupe hydroxyalkyle en C₁-C₄ ou un groupe polyhydroxyalkyle en C₂-C₄,
R² représente un atome d'hydrogène ou un groupe -COOH, le groupe COOH pouvant être également présent sous la forme d'un sel comprenant un cation physiologiquement compatible,
R³ représente un atome d'hydrogène ou un groupe alkyle en C₁-C₄,
R⁴ représente un atome d'hydrogène, un groupe alkyle en C₁-C₄ ou un groupe -CO-R⁶ dans lequel
R⁶ représente un groupe alkyle en C₁-C₄, et
R⁵ représente l'un des groupes mentionnés sous R⁴
ou un sel physiologiquement compatible de ces composés.

3. Agent selon la revendication 2, **caractérisé en ce que** le composé répondant à la formule (1a) est choisi dans le groupe constitué de la 5,6-dihydroxyindoline, de la N-méthyl-5,6-dihydroxyindoline, de la N-éthyl-5,6-dihydroxyindoline, de la N-propyl-5,6-dihydroxyindoline, de la N-butyl-5,6-dihydroxyindoline et de l'acide 5,6-dihydroxyindolin- 2-carboxylique.

4. Agent selon la revendication 3, **caractérisé en ce que** le composé répondant à la formule (1a) est la 5,6-dihydroxyindoline.

5. Agent selon l'une des revendications 1 à 4, **caractérisé en ce qu'**il contient comme composant (A) au moins un dérivé d'indoline répondant à la formule (1 b), 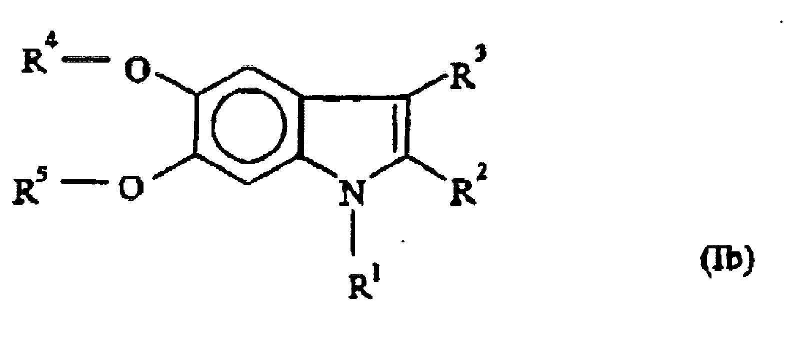 dans laquelle indépendamment les uns des autres
R¹ représente un atome d'hydrogène, un groupe alkyle en C₁-C₄, un groupe hydroxyalkyle en C₁-C₄ ou un groupe polyhydroxyalkyle en C₂-C₄,
R² représente un atome d'hydrogène ou un groupe -COOH, le groupe -COOH pouvant être également présent sous la forme d'un sel comprenant un cation physiologiquement compatible,
R³ représente un atome d'hydrogène ou un groupe alkyle en C₁-C₄,
R⁴ représente un atome d'hydrogène, un groupe alkyle en C₁-C₄ ou un groupe - CO-R⁶, dans lequel R⁶ représente un groupe alkyle en C₁-C₄, et
R⁵ représente l'un des groupes mentionnés sous R⁴
de même que l'un des sels physiologiquement compatibles de ces composés.

6. Agent selon la revendication 5, **caractérisé en ce que** le dérivé d'indole répondant à la formule (1 b) est choisi dans le groupe constitué du 5,6-dihydroxyindole, du N-méthyl-5,6-dihydroxyindole, du N-éthyl-5,6-dihydroxyindole, du N-propyl-5,6-dihydroxyindole, du N-butyl-5,6-dihydroxyindole et de l'acide 5,6-dihydroxyindol-2-carboxylique.

7. Agent selon la revendication 6, **caractérisé en ce que** le dérivé d'indole répondant à la formule (1 b) est le 5,6-dihydroxyindole.

8. Agent selon l'une des revendications 1 à 7, **caractérisé en ce que** le dérivé de pyrimidine est choisi dans le groupe constitué de la 2,4,5,6-tétraaminopyrimidine, de la 2-diméthylamino-4,5,6-triaminopyrimidine, de la 2-hydroxy-4,5,6-triaminopyrimidine et de la 4-hydroxy-2,5,6-triaminopyrimidine.

9. Agent selon la revendication 8, **caractérisé en ce que** le dérivé de pyrimidine est la 2,4,5,6-tétraaminopyrimidine.

10. Agent selon l'une des revendications 1 à 9, **caractérisé en ce qu'**il contient au moins un composant révélateur supplémentaire.

11. Agent selon l'une des revendications 1 à 10, **caractérisé en ce que** le dérivé de résorcinol est choisi dans le groupe constitué du résorcinol, du monométhyléther de résorcinol, du 2-méthylrésorcinol, du 5-méthylrésorcinol, du 2,5-diméthylrésorcinol, du 2-chlororésorcinol, du 4-chlororésorcinol, du pyrogallol et du 1,2,4-trihydroxybenzène.

12. Agent selon la revendication 11, **caractérisé en ce que** le dérivé de résorcinol est le 2-méthylrésorcinol.

13. Agent selon l'une des revendications 1 à 12, **caractérisé en ce qu'**il contient en outre au moins un colorant montant directement sur la fibre.

14. Utilisation d'un agent selon l'une des revendications 1 à 13 pour la teinture de fibres kératiniques.

15. Utilisation selon la revendication 14 pour accroître la résistance au lavage de la teinture résultante - en particulier dans le domaine des nuances de rouge.

16. Procédé de teinture de fibres kératiniques, dans lequel un agent contenant
(A) au moins un dérivé d'indole et/ou d'indoline,
(B) au moins un dérivé de pyrimidine et
(C) comme composant de couplage, au moins un dérivé de résorcinol
est appliqué sur les fibres et est éliminé par rinçage après une certaine durée d'action.

17. Procédé selon la revendication 16, **caractérisé en ce que** l'agent contient en outre un agent d'oxydation, particulièrement le peroxyde d'hydrogène.
